(19) Europäisches Patentamt
European Patent Office
Office européen des brevets

(11) EP 0 994 838 B1

(12) **FASCICULE DE BREVET EUROPEEN**

(45) Date de publication et mention de la délivrance du brevet:
**21.05.2003 Bulletin 2003/21**

(21) Numéro de dépôt: **98936479.9**

(22) Date de dépôt: **08.07.1998**

(51) Int Cl.[7]: **C07C 41/09**, C11B 9/00, C07C 43/23, C07C 43/178, C07C 43/205

(86) Numéro de dépôt international:
**PCT/FR98/01472**

(87) Numéro de publication internationale:
**WO 99/002475 (21.01.1999 Gazette 1999/03)**

(54) **PROCEDE D'ETHERIFICATION D'UN ALCOOL DE TYPE BENZYLIQUE, PRODUITS OBTENUS ET SES APPLICATIONS**

VERFAHREN ZUR ETHERIFIZIERUNG VON BENZYLALKOHOLEN, ERHALTENE PRODUKTE UND IHRE VERWENDUNGEN

METHOD FOR ETHERIFYING A BENZYL ALCOHOL, RESULTING PRODUCTS AND APPLICATIONS

(84) Etats contractants désignés:
**AT BE CH DE DK ES FI FR GB GR IE IT LI LU NL PT SE**

(30) Priorité: **09.07.1997 FR 9708733**

(43) Date de publication de la demande:
**26.04.2000 Bulletin 2000/17**

(73) Titulaire: **RHODIA CHIMIE**
**92408 Courbevoie Cédex (FR)**

(72) Inventeurs:
- **JACQUOT, Roland**
**F-69110 Sainte Foy lès Lyon (FR)**
- **SPAGNOL, Michel**
**F-69008 Lyon (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:

| | |
|---|---|
| **EP-A- 0 137 419** | **EP-A- 0 258 172** |
| **WO-A-95/00467** | **CH-A- 681 778** |
| **DE-A- 3 502 188** | **DE-A- 4 434 823** |
| **DE-C- 154 658** | **US-A- 4 147 671** |

- **"Perfume and Flavor Chemicals" 1969 , ARCTANDER , MONTCLAIR, N. J. XP002081064 voir nos. 311, 312, 313, 336, 337, 1919**
- **H. BOHNSACK: "Beitrag zur Kenntnis der ätherischen Oele, Riech- und Geschmackstoffe XV. Mitteilung: Ueber die Inhaltstoffe der Bourbon-Vanilleschote III. Teil: Vanillylalkohol und andere aromatische Hydroxyalkohole bzw. den Methylaetherer" RIECHSTOFFE, AROMEN, KÖRPERPFLEGEMITTEL, vol. 15, no. 11, novembre 1965, pages 407-410, XP002081410 Hannover**
- **M. J. CLIMENT: "Hydride transfer reactions of benzylic alcohols catalyzed by acid faujasites" RECUEIL DES TRAVAUX CHIMIQUES DES PAYS-BAS, vol. 110, no. 6, juin 1991, pages 275-278, XP002056679 AMSTERDAM NL**
- **T. M. WORTEL: "Zeolite catalyzed liquid phase dehydration of alpha-phenylethanols" ACTA PHYS. CHEM., vol. 24, no. 1-2, 1978, pages 341-346, XP002056680**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 994 838 B1

## Description

La présente invention a pour objet un procédé d'éthérification d'un alcool de type benzylique, les produits obtenus et leurs applications notamment dans le domaine de la parfumerie.

Il est connu de préparer selon DE-A-4434823 des éthers d'alcools hydroxybenzyliques par réaction de l'alcool hydroxybenzylique et d'un alcanol, en présence d'une résine sulfonique (Amberlyst® A21). La résine est difficilement régénérable et le rendement réactionnel obtenu n'est pas très satisfaisant puisque seulement de 72 %.

Le procédé décrit n'est pas compatible avec une exploitation industrielle.

La présente invention a précisement pour but de proposer un procédé permettant d'obvier ces inconvénients.

Il a maintenant été trouvé et c'est ce qui constitue l'objet de la présente invention, un procédé d'éthérification d'un alcool de type benzylique qui consiste à faire réagir ledit alcool avec un autre alcool, en présence d'un catalyseur, ledit procédé étant caractérisé par le fait que l'on conduit la réaction d'éthérification en présence d'une quantité efficace d'une zéolithe.

Dans l'exposé qui suit de la présente invention, on entend "par alcool de type benzylique", un carbocycle ou un hétérocycle aromatique dont un atome d'hydrogène directement lié au noyau aromatique est remplacé par un groupe

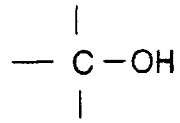

et par "aromatique", la notion classique d'aromaticité telle que définie dans la littérature, notamment par Jerry MARCH, Advanced Organic Chemistry, 4ème édition, John Wiley and Sons, 1992, pp. 40 et suivantes.

Par commodité de langage, l'autre alcool mis en oeuvre sera dénommée de manière générique par "alcanol" bien qu'il désigne également des alcools comportant des cycles notamment aromatiques.

Plus précisément, la présente invention a pour objet un procédé d'éthérification d'un alcool benzylique de formule générale (I) :

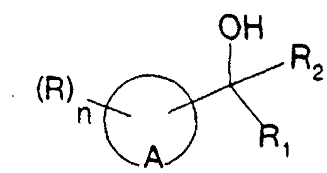
(I)

dans laquelle :

- A symbolise le reste d'un cycle formant tout ou partie d'un système carbocyclique ou hétérocyclique, aromatique, monocyclique ou polycyclique, système comprenant au moins un groupe

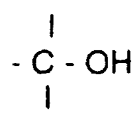

- R représente un ou plusieurs substituants, identiques ou différents,
- $R_1$ et $R_2$, identiques ou différents, représentent un atome d'hydrogène, un groupe fonctionnel ou un groupe hydrocarboné ayant de 1 à 24 atomes de carbone, qui peut être un groupe aliphatique acyclique saturé ou insaturé, linéaire ou ramifié ; un groupe cycloaliphatique saturé, insaturé ou aromatique, monocyclique ou polycyclique ; un groupe aliphatique saturé ou insaturé, linéaire ou ramifié, porteur d'un substituant cyclique,
- $R_1$ et $R_2$ peuvent former un cycle comprenant éventuellement un autre hétéroatome,
- n est un nombre inférieur ou égal à 5.

L'alcool de type benzylique qui intervient dans le procédé de l'invention répond à la formule (I) dans laquelle $R_1$ et $R_2$ représentent un groupe aliphatique acyclique, saturé ou insaturé, linéaire ou ramifié.

Plus préférentiellement; $R_1$ et $R_2$ représentent un groupe alkyle linéaire ou ramifié ayant de 1 à 12 atomes

de carbone, de préférence de 1 à 6 atomes de carbone : la chaîne hydrocarbonée pouvant être éventuellement interrompue par un hétéroatome (par exemple, l'oxygène), par un groupe fonctionnel (par exemple -CO-) et/ou porteuse d'un substituant (par exemple, un halogène).

Le groupe aliphatique acyclique, saturé ou insaturé, linéaire ou ramifié peut être éventuellement porteur d'un substituant cyclique. Par cycle, on entend de préférence, un cycle carbocyclique ou hétérocyclique, saturé, insaturé ou aromatique, de préférence cycloaliphatique ou aromatique notamment cycloaliphatique comprenant 6 atomes de carbone dans le cycle ou benzénique.

Le groupe aliphatique acyclique peut être relié au cycle par un lien valentiel, un hétéroatome ou un groupe fonctionnel et des exemples sont donnés ci-dessus.

Le cycle peut être éventuellement substitué et à titre d'exemples de substituants cycliques, on peut envisager, entre autres, les substituants tels que R dont la signification est précisée ci-après.

$R_1$ et $R_2$ peuvent représenter également un groupe carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué par des substituants tels que R.

$R_1$ et $R_2$ peuvent représenter également un groupe carbocyclique aromatique, de préférence monocyclique ayant généralement au moins 4 atomes de carbone, de préférence, 6 atomes de carbone dans le cycle ; ledit cycle pouvant être substitué par des substituants tels que R.

L'un des groupes $R_1$ et $R_2$ peut représnter un groupe $CF_3$.

Dans la formule (I), les groupes $R_1$ et $R_2$, peuvent former entre eux, un cycle ayant de préférence, de 5 à 7 atomes, saturé ou insaturé, comprenant éventuellement un autre hétéroatome, par exemple un atome d'oxygène.

L'invention s'applique notamment aux alcools de type benzylique répondant à la formule (I) dans laquelle A est le reste d'un composé cyclique, ayant de préférence, au moins 4 atomes dans le cycle, de préférence, 5 ou 6, éventuellement substitué, et représentant au moins l'un des cycles suivants :

- un carbocycle aromatique, monocyclique ou polycyclique,
- un hétérocycle aromatique, monocyclique ou polycyclique comportant au moins un des hétéroatomes O, N et S,

On précisera, sans pour autant limiter la portée de l'invention, que le reste A éventuellement substitué représente, le reste :

- d'un composé monocyclique, carbocyclique, aromatique, tel que par exemple, le benzène ou le toluène,
- d'un composé polycyclique, condensé, aromatique, tel que par exemple, le naphtalène,
- d'un composé monocyclique, hétérocyclique, aromatique, tel que par exemple, la pyridine, le furane, le thiophène.

Dans le procédé de l'invention, on met en oeuvre préférentiellement un composé aromatique de formule (I) dans laquelle A représente un noyau benzénique ou naphtalénique.

Le reste A de l'alcool de type benzylique de formule (I) peut être porteur d'un ou plusieurs substituants.

Le nombre de substituants présents sur le cycle dépend de la condensation en carbone du cycle et de la présence ou non d'insaturations sur le cycle.

Le nombre maximum de substituants susceptibles d'être portés par un cycle, est aisément déterminé par l'Homme du Métier.

Dans le présent texte, on entend par "plusieurs", généralement, moins de 5 substituants sur un noyau aromatique.

Des exemples de substituants sont donnés ci-dessous mais cette liste ne présente pas de caractère limitatif. On peut citer notamment :

- les groupes alkyle linéaires ou ramifiés ayant de préférence de 1 à 6 atomes de carbone et encore plus préférentiellement de 1 à 4 atomes de carbone,
- les groupes alcényle linéaires ou ramifiés ayant de préférence de 2 à 6 atomes de carbone et encore plus préférentiellement de 2 à 4 atomes de carbone,
- les groupes halogénoalkyle linéaires ou ramifiés ayant de préférence de 1 à 6 atomes de carbone et encore plus préférentiellement de 1 à 4 atomes de carbone,
- les groupes cycloalkyle ayant de 3 à 6 atomes de carbone, de préférence, le groupe cyclohexyle,
- le groupe phényle,
- le groupe hydroxyle,
- le groupe $NO_2$,
- les groupes alkoxy $R_3$-O- ou thioéther $R_3$-S- dans lesquels $R_3$ représente un groupe alkyle linéaire ou ramifié ayant de 1 à 6 atomes de carbone et encore plus préférentiellement de 1 à 4 atomes de carbone ou le groupe phényle,

- les groupes $-N-(R_4)_2$ dans lesquels les groupes $R_4$ identiques ou différents représentent un atome d'hydrogène, un groupe alkyle linéaire ou ramifié ayant de 1 à 6 atomes de carbone et encore plus préférentiellement de 1 à 4 atomes de carbone ou un groupe phényle,
- les groupes $-NH-CO-R_4$ dans lesquels le groupe $R_4$ a la signification donnée précédemment,
- les groupes carboxy ou dérivé $R_4$-O-CO- dans lesquels le groupe $R_4$ a la signification donnée précédemment,
- les groupes acyloxy ou aroyloxy $R_3$-CO-O- dans lesquels le groupe $R_3$ a la signification donnée précédemment,
- un atome d'halogène, de préférence, un atome de fluor,
- un groupe $CF_3$.

Lorsque n est supérieur ou égal à 2, deux groupes R et les 2 atomes successifs du cycle aromatique peuvent être liés entre eux par un groupe alkylène, alcénylène ou alcénylidène ayant de 2 à 4 atomes de carbone pour former un hétérocycle saturé, insaturé ou aromatique ayant de 5 à 7 atomes de carbone. Un ou plusieurs atomes de carbone peuvent être remplacés par un autre hétéroatome, de préférence l'oxygène. Ainsi, les groupes R peuvent représenter un groupe méthylènedioxy ou éthylènedioxy.

Les substituants préférés sont choisis parmi les groupes électro-donneurs.

On entend par "groupe électro-donneur", un groupe tel que défini par H.C. BROWN dans l'ouvrage de Jerry MARCH - Advanced Organic Chemistry, chapitre 9, pages 243 et 244 (1985).

Le procédé de l'invention s'applique tout particulièrement aux alcools de type benzylique de formule (Ia) :

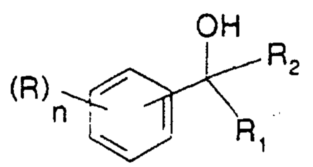

(Ia)

dans laquelle :

- n est un nombre inférieur ou égal à 4, de préférence égal à 0, 1 ou 2,
- le groupe R est un groupe électro-donneur, de préférence, un groupe alkyle, alkoxy ou méthylènedioxy ou éthylènedioxy,
- les groupes $R_1$ et $R_2$, identiques ou différents, représentent :

  . un atome d'hydrogène,
  . un groupe alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un groupe cycloalkyle ayant de 3 à 8 atomes de carbone, de préférence, un groupe cyclopentyle ou cyclohexyle,
  . un groupe phényle,
  . un groupe phénylalkyle ayant de 7 à 12 atomes de carbone, de préférence, un groupe benzyle,
  . un groupe $CF_3$.

Les composés préférés répondent à la formule (Ia) dans laquelle :

- n est un nombre égal à 0, 1 ou 2,
- les groupes R, identiques ou différents, représentent un groupe alkyle, alkoxy ou méthylènedioxy ou éthylène dioxy, hydroxyle,
- les groupes $R_1$ et $R_2$, identiques ou différents, représentent :

  . un atome d'hydrogène,
  . un groupe alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle.

Les alcools de type benzylique mis en oeuvre préférentiellement dans le procédé de l'invention sont :

- l'alcool vanillique,
- l'alcool p-hydroxybenzylique,
- le 1-(4-hydroxy-3-méthoxyphényl)éthanol,

- l'alcool 2-hydroxybenzylique,
- l'alcool p-méthoxybenzylique,
- l'alcool 3,4-diméthoxybenzylique,
- l'alcool 6-n-propyl-3,4-diméthoxybenzylique,
- l'alcool (3,4-diméthoxyphényl)diméthylcarbinol,
- le 1-[1-hydroxy-2-méthylpropyl]-3,4-diméthoxybenzène,
- le 1-[1-hydroxy-2-méthylpropyl]-3,4-diéthoxybenzène,
- le 1-[1-hydroxyéthyl]-3,4-diéthoxybenzène,
- le 1-[1-hydroxyéthyl]-3,4-diméthoxy-6-propylbenzène,
- le 5-[1-hydroxyéthyl]-1,3-benzodioxol,
- le naphtalène-2-méthylol.

Pour ce qui est de l'alcanol, il répond plus particulièrement à la formule générale (II) :

$$R_5 - OH \qquad (II)$$

dans ladite formule (II) :

- $R_5$ représente un groupe hydrocarboné ayant de 1 à 24 atomes de carbone, qui peut être un groupe aliphatique acyclique saturé ou insaturé, linéaire ou ramifié ; un groupe cycloaliphatique saturé, insaturé ou aromatique, un groupe aliphatique saturé ou insaturé, linéaire ou ramifié, porteur d'un substituant cyclique.

L'alcanol qui intervient dans le procédé de l'invention répond à la formule (II) dans laquelle $R_5$ représente un groupe aliphatique acyclique, saturé ou insaturé, linéaire ou ramifié.
Plus précisément, $R_5$ représente un groupe alkyle, alcényle, alcadiényle, alcynyle, linéaire ou ramifié ayant de préférence de 1 à 24 atomes de carbone.
La chaîne hydrocarbonée peut être éventuellement :

- interrompue par l'un des groupes suivants :

  $-O-, -CO-, -COO-, -OCOO-, -S-, -SO_2-,$

  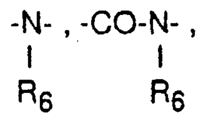

  dans ces formules $R_6$ représente l'hydrogène ou un groupe alkyle linéaire ou ramifié ayant de 1 à 4 atomes de carbone, de préférence, un groupe méthyle ou éthyle,
- et/ou porteuse de l'un des substituants suivants :

  $-OH, -OCOO-, -COOR_6, -CHO, -NO_2, -X, -CF_3$

  dans ces formules, $R_6$ ayant la signification donnée précédemment.

Le reste aliphatique acyclique, saturé ou insaturé, linéaire ou ramifié peut être éventuellement porteur d'un substituant cyclique. Par cycle, on entend un cycle carbocyclique ou hétérocyclique, saturé, insaturé ou aromatique.
Le reste aliphatique acyclique peut être relié au cycle par un lien valentiel ou par l'un des groupes suivants :

$-O-, -CO-, -COO-, -OCOO-, -S-, -SO_2-,$

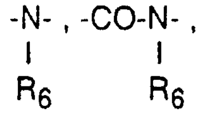

dans ces formules, $R_6$ ayant la signification donnée précédemment.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs de 1, 2, 3, 4 ou 5 groupes R', identiques ou différents, R' ayant la signification donnée précédemment pour le groupe R porté par le cycle de la formule (I).

Dans la formule générale (II) des alcanols, $R_5$ peut représenter également un groupe carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 groupes R' de préférence 1 à 3, R' ayant les significations énoncées précédemment pour R.

Comme exemples préférés de groupes $R_5$, on peut citer les groupes cyclohexyle ou cyclohexène-yle, éventuellement substitués par des groupes alkyle linéaires ou ramifiés, ayant de 1 à 4 atomes de carbone.

Le procédé est mis en oeuvre aisément avec la plupart des alcanols. Comme exemples d'alcanols, on peut citer :

- les alcanols aliphatiques inférieurs ayant de 1 à 5 atomes de carbone, tels que par exemple, le méthanol, l'éthanol, le trifluoroéthanol, le propanol, l'alcool isopropylique, le butanol, l'alcool isobutylique, l'alcool sec-butylique, l'alcool tert-butylique, le pentanol, l'alcool isopentylique, l'alcool sec-pentylique et l'alcool tert-pentylique, l'éther monoéthylique d'éthylèneglycol, le lactate de méthyle, le lactate d'isobutyle, le D-lactate de méthyle, le D-lactate d'isobutyle, le 3-chlorobut-2-én-1-ol, le 2-butyn-1-ol,
- les alcools aliphatiques supérieurs ayant au moins 6 et jusqu'à environ 20 atomes de carbone, tels que par exemple, l'hexanol, l'heptanol, l'alcool isoheptylique, l'octanol, l'alcool isooctylique, l'éthyl-2 hexanol, l'alcool secoctylique, l'alcool tert-octylique, le nonanol, l'alcool isononylique, le décanol, le dodécanol, le tétradécanol, l'octadécanol, l'hexadécanol, l'alcool oléylique, l'alcool eicosylique, l'éther monoéthylique de diéthylèneglycol.
- les alcools cycloaliphatiques ayant de 3 à environ 20 atomes de carbone, tel que, par exemple, le cyclopropanol, le cyclobutanol, le cyclopentanol, le cyclohexanol, le cycloheptanol, le cyclooctanol, le cyclododécanol, le tripropylcyclohexanol, le méthylcyclohexanol et le méthylcycloheptanol, le cyclopentèn-ol, le cyclohexèn-ol,
- un alcool aliphatique porteur d'un groupement aromatique ayant de 7 à environ 20 atomes de carbone tel que par exemple l'alcool benzylique, l'alcool phénéthylique, l'alcool phénylpropylique, l'alcool phényloctadécylique et l'alcool naphtyldécylique.

Il est également possible de mettre en oeuvre des polyols notamment les polyoxyéthylèneglycols tels que, par exemple, l'éthylène glycol, le diéthylène glycol, le triéthylène glycol, le propylène glycol, le glycérol.

Parmi les alcools précités, on met en oeuvre de préférence dans le procédé de l'invention, les alcools aliphatiques ou cycloaliphatiques, de préférence, les alcools aliphatiques primaires ou secondaires ayant 1 à 4 atomes de carbone et le cyclohexanol.

Une variante préférée du procédé de l'invention consiste à mettre en oeuvre un alcool terpénique et plus particulièrement un alcool terpénique de formule (IIa) :

$$T - OH \qquad (IIa)$$

dans ladite formule (IIa) :

- T représente le reste d'un alcool terpénique ayant un nombre d'atomes de carbone multiple de 5.

Dans l'exposé qui suit de la présente invention, on entend par "terpène", les oligomères dérivés de l'isoprène.

Plus précisément, l'alcool mis en oeuvre répond à la formule générale (IIa) dans laquelle le reste T représente un groupe hydrocarboné ayant de 5 à 40 atomes de carbone et plus particulièrement un groupe aliphatique, saturé ou insaturé, linéaire ou ramifié ; un groupe cycloaliphatique, saturé, insaturé ou aromatique, monocyclique ou polycyclique, comprenant des cycles ayant de 3 à 8 atomes de carbone.

On précisera, sans pour autant limiter la portée de l'invention, que le reste T représente, le reste :

- d'un alcool terpénique aliphatique, saturé ou insaturé, linéaire ou ramifié,
- d'un alcool terpénique cycloaliphatique, monocyclique, saturé ou insaturé, ou aromatique,
- d'un alcool terpénique cycloaliphatique, polycyclique comprenant au moins deux carbocycles, saturés et/ou insaturés.

S'agissant du reste T d'un alcool terpénique aliphatique, saturé ou insaturé, linéaire ou ramifié, le nombre d'atomes de carbone varie entre 5 et 40 atomes de carbone. Comme exemples plus spécifiques de reste T, on peut mentionner les groupes comprenant 8 atomes de carbone, saturé ou présentant une double liaison, et portant deux groupes méthyle, de préférence en position 3 et 7.

Lorsqu'il s'agit d'un composé monocyclique, le nombre d'atomes de carbone dans le cycle peut varier largement de 3 à 8 atomes de carbone mais il est de préférence de 5 ou 6 atomes de carbone et est le plus souvent porté par une chaîne aliphatique.

Le carbocycle peut être saturé ou comprenant 1 ou 2 insaturations dans le cycle, de préférence de 1 à 2 doubles liaisons qui sont le plus souvent en position $\alpha$ de l'atome d'oxygène.

Dans le cas d'un alcool terpénique aromatique, le cycle aromatique est généralement un noyau benzénique.

Le composé peut être également polycyclique, de préférence bicyclique ce qui signifie qu'au moins deux cycles ont deux atomes de carbone en commun. Dans le cas des composés polycycliques, le nombre d'atomes de carbone dans chaque cycle varie entre 3 et 6 : le nombre total d'atomes de carbone étant égal de préférence à 7.

On donne ci-après des exemples de structure bicyclique, couramment rencontrée :

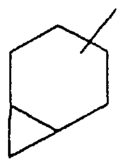
[4,1,0]

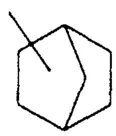
[2,2,1]

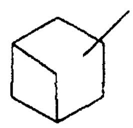
[3,1,1]

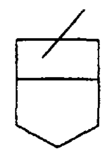
[3,2,0]

Dans le cas d'un cycle, la présence de substituants n'est pas exclue dans la mesure où ils sont compatibles avec l'application envisagée. Les substituants portés le plus souvent par le carbocycle sont un ou plusieurs groupes alkyle, de préférence trois groupes méthyle, un groupe méthylène (correspondant à une liaison exocyclique), un groupe alcényle, de préférence un groupe isopropène-yle.

Comme exemples d'alcools terpéniques susceptibles d'être mis en oeuvre, on peut citer :

- les alcools terpéniques aliphatiques saturés ou insaturés tels que :
  - . le 3,7-diméthyloctanol,
  - . le tétrahydro-allociménol,
  - . l'hydroxycitronellol,
  - . le 1-hydroxy 3,7-diméthyl 7-octène,
  - . le nérol,
  - . le géraniol,
  - . le linalool,
  - . le citronellol,
  - . le 3,7-diméthyloct-6-én-1-ol
  - . le 1-hydroxy 2-éthyl 5-isopropyl 8-méthyl 2,7-nonadiène,
  - . le 1-hydroxy 3,7,11-triméthyl 6,10-dodécadiène,
- les alcools terpéniques cycloaliphatiques aromatiques tels que :
  - . le thymol,
- les alcools terpéniques cycloaliphatiques monocycliques ou polycycliques, saturés ou insaturés tels que :
  - . l'alcool chrysanthémique,
  - . le 1-hydroxyéthyl 2,2,3-triméthylcyclopentane,
  - . l'hydrate de terpinol,

- . le 1,8-terpine,
- . le dihydro-terpinéol,
- . le β-terpinéol,
- . l'alcool périllylique,
- . le 1-méthyl 3-hydroxy 4-isopropylcyclohexène,
- . l'α-terpinéol,
- . le terpinène-4-ol,
- . le 1,3,5-triméthyl 4-hydroxyméthylcyclohexène,
- . le carvéol,
- . le cis-2-pinanol,
- . le cis-3-pinanol,
- . l'isobornéol,
- . le verbénol,
- . le trans-pinocarvéol,
- . l'alcool campholénique,
- . le (diméthyl-2,3-tricyclo-[2.2.1.0(2,6)]-heptyl-3)-5-méthyl-2-pentène-2-ol-1 ou santalol.

Parmi tous les alcools précités, les alcools préférés sont les suivants :

- . l'alcool chrysantémique,
- . le 3,7-diméthyloctanol,
- . le géraniol,
- . le linalool,
- . le citronellol,
- . l'hydoxycitronellol,
- . le nérol,
- . le thymol,
- . le menthol,
- . l'isobornéol,
- . le verbénol.

Conformément au procédé de l'invention, on effectue la réaction d'hydroxyalkylation en présence d'un catalyseur constitué par une zéolithe.

Par "zéolithe", on entend un tectosilicate cristallisé d'origine naturelle ou synthétique dont les cristaux résultent de l'assemblage tridimensionnel d'unités tétraédriques de $SiO_4$ et $TO_4$ : T représentant un élément trivalent tel que aluminium, gallium, bore, fer, de préférence, l'aluminium.

Les zéolithes de type aluminosilicate sont les plus communes.

Les zéolithes présentent au sein du réseau cristallin, un système de cavités reliées entre elles par des canaux d'un diamètre bien défini que l'on appelle les pores.

Les zéolithes peuvent présenter un réseau de canaux monodimensionnel, bidimensionnel ou tridimensionnel.

Dans le procédé de l'invention, on peut faire appel à une zéolithe naturelle ou synthètique.

Comme exemples de zéolithes naturelles susceptibles d'être utilisées, on peut citer, par exemple : la chabazite, la clinoptilolite, l'érionite, la phillipsite, l'offrétite.

Conviennent tout à fait bien à la mise en oeuvre de l'invention, les zéolithes synthétiques.

Comme exemples de zéolithes synthétiques à réseau monodimensionnel, on peut citer entre autres, la zéolithe ZSM-4, la zéolithe L, la zéolithe ZSM-12, la zéolithe ZSM-22, la zéolithe ZSM-23, la zéolithe ZSM-48.

A titre d'exemples de zéolithes à réseau bidimensionnel mises en oeuvre préférentiellement, on peut mentionner la mordénite, la ferrierite.

En ce qui concerne les zéolithes à réseau tridimensionnel, on peut nommer plus particulièrement, la zéolithe β, la zéolithe Y, la zéolithe X, la zéolithe ZSM-5, la zéolithe ZSM-11, l'offrétite.

On fait appel préférentiellement aux zéolithes synthétiques et plus particulièrement aux zéolithes qui sont sous les formes suivantes :

- la mazzite de rapport molaire Si/Al de 3,4,
- la zéolithe L de rapport molaire Si/Al de 1,5 à 3,5,
- la mordénite de rapport molaire Si/Al de 5 à 150, de préférence, de 10 à 100 et encore plus préférentiellement de 10 à 25,
- la ferrierite de rapport molaire Si/Al de 3 à 10,

- l'offrétite de rapport molaire Si/Al de 4 à 8,5.
- les zéolithes β de rapport molaire Si/Al supérieur à 8, de préférence entre 10 à 100, et encore plus préférentiellement de 12 à 50,
- les zéolithes Y en particulier les zéolithes obtenues après traitement de désalumination (par exemple hydrotraitement, lavage à l'aide d'acide chlorhydrique ou traitement par $SiCl_4$) et l'on peut citer plus particulièrement les zéolithes US-Y de rapport molaire Si/Al supérieur à 3, de préférence compris entre 6 et 60 ;
- la zéolithe X de type faujasite de rapport molaire Si/Al de 0,7 à 1,5,
- les zéolithes ZSM-5 ou silicalite d'aluminium de rapport molaire Si/Al de 10 à 500,
- la zéolithe ZSM-11 de rapport molaire Si/Al de 5 à 30.
- la zéolithe ou solide mésoporeux de type MCM, plus particulièrement MCM-22 et MCM-41 de rapport molaire Si/Al compris entre 10 et 100, de préférence, compris entre 15 et 40.

Parmi toutes ces zéolithes, on fait appel préférentiellement dans le procédé de l'invention aux zéolithes β.

Les zéolithes mises en oeuvre dans le procédé de l'invention, sont des produits connus décrits dans la littérature [cf. Atlas of zeolites structure types by W. M. Meier and D. H. Olson published by the Structure Commission of the international Zeolite Association (1992)].

On peut faire appel aux zéolithes disponibles dans le commerce ou bien les synthétiser selon les procédés décrits dans la littérature.

On peut se référer à l'Atlas précité, et plus particulièrement, pour la préparation :

- de la zéolithe L à la publication de Barrer R. M. et al, Z. Kristallogr., 128, pp. 352 (1969)
- de la zéolithe ZSM-12, au brevet US 3 832 449 et à l'article LaPierre et al, Zeolites 5, pp. 346 (1985),
- de la zéolithe ZSM-22, à la publication Kokotailo G.T. et al, Zeolites 5, pp. 349 (1985),
- de la zéolithe ZSM-23, au brevet US 4 076 842 et à l'article Rohrman A. C. et al, Zeolites 5, pp. 352 (1985),
- de la zéolithe ZSM-48, aux travaux de Schlenker J. L. et al, Zeolites 5, pp. 355 (1985),
- de la zéolithe β, au brevet US 3 308 069 et à l'article Caullet P. et al, Zeolites 12, pp. 240 (1992),
- de la mordénite, aux travaux de Itabashi et al, Zeolites 6, pp. 30 (1986),
- des zéolithes X et Y respectivement aux brevets US 2 882 244 et US 3 130 007,
- de la zéolithe ZSM-5, au brevet US 3 702 886 et à l'article Shiralkar V. P. et al, Zeolites 9, pp. 363 (1989),
- de la zéolithe ZSM-11, aux travaux de Harrison I. D. et al, Zeolites 7, pp. 21 (1987).
- de la zéolithe ou solide mésoporeux MCM à l'article de Beck et al, J. Am. Chem. Soc. 114(27), pp. 10834-43 (1992).

Afin de mettre en oeuvre une zéolithe présentant le rapport atomique Si/Al souhaité, il peut être nécessaire d'effectuer un traitement de désalumination.

Ainsi, on peut mettre en oeuvre les méthodes connues de l'homme du métier parmi lesquelles on peut citer, à titre d'exemples, et de manière non exhaustive, les calcinations en présence de vapeur, les calcinations en présence de vapeur d'eau suivies d'attaques par des acides minéraux ($HNO_3$, HCl...), les traitements directes de désalumination par des réactifs tels que le tétrachlorure de silicium ($SiCl_4$), l'hexafluorosilicate d'ammonium ($(NH_4)_2SiF_6$), l'éthylènediaminetétracétique (EDTA) ainsi que sa forme mono- ou disodique. On peut également faire un traitement de désalumination par attaque acide directe par des solutions d'acides minéraux tels que par exemple, l'acide chlorhydrique, l'acide nitrique, l'acide sulfurique ou d'acides organiques comme notamment l'acide acétique, l'acide oxalique.
Par ailleurs, toute combinaison des méthodes de désalumination précitées est aussi possible.

La zéolithe constitue la phase catalytique. Elle peut être utilisée seule ou en mélange avec une matrice minérale. Dans la description, on désignera par "catalyseur", le catalyseur réalisé entièrement en zéolithe ou en mélange avec une matrice préparée selon des techniques connues de l'Homme du métier.

A cet effet, la matrice peut être choisie parmi les oxydes de métaux, tels que les oxydes d'aluminium, de silicium et/ou de zirconium, ou encore parmi les argiles et plus particulièrement, le kaolin, le talc ou la montmorillonite.

Dans le catalyseur, la teneur en phase active représente de 5 à 100 % du poids du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

La zéolithe mise en oeuvre est de préférence, sous forme acide. On effectue si nécessaire un traitement qui la rend acide.

A cet effet, on fait appel aux traitements classiques.

Ainsi, on peut échanger les cations alcalins en soumettant la zéolithe à un traitement réalisé avec de l'ammoniaque conduisant ainsi à un échange du cation alcalin par un ion ammonium puis à calciner la zéolithe échangée

afin de décomposer thermiquement le cation ammonium et le remplacer par un ion $H^+$.

La quantité d'ammoniaque à mettre en oeuvre est au moins égale à la quantité nécessaire pour échanger tous les cations alcalins en ions $NH_4^+$.

On met donc au moins en jeu de $10^{-5}$ à $5.10^{-3}$ mole d'ammoniaque par gramme de zéolithe.

La réaction d'échange du cation échangeable par $NH_4^+$ est effectuée à une température qui se situe entre la température ambiante et la température de reflux du milieu réactionnel. L'opération dure quelques heures et peut être répétée.

La zéolithe peut être également acidifiée en soumettant celle-ci à un traitement acide classique. Ce traitement peut être effectué par addition d'un acide tel que notamment l'acide chlorhydrique, l'acide sulfurique, l'acide nitrique, l'acide perchlorique, l'acide phosphorique et l'acide trifluorométhanesulfonique.

Selon un mode préférentiel de mise en oeuvre, la zéolithe est acidifiée par passage d'un volume d'acide présentant une normalité comprise entre 0,1 et 2 N par gramme de zéolithe comprise entre 10 ml/g et 100 ml/g. Ce passage peut être réalisé en une seule étape ou de préférence en plusieurs étapes successives.

La réaction de l'alcool benzylique de formule (i) avec l'alcanol de formulé (II) peut être conduite en présence ou en l'absence d'un solvant organique, l'un des réactifs pouvant être utilisé comme solvant réactionnel.

Comme exemples de solvants convenant à la présente invention, on peut citer, sans caractère limitatif, les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, l'oxyde de phényle, l'oxyde de benzyle ; le dioxane, le tétrahydrofuranne (THF).

Lorsque l'on réalise le procédé en discontinu, le catalyseur peut représenter en poids par rapport au réactif en défaut, de 2 à 50 %, de préférence, de 5 à 20 %. Cependant si l'on réalise le procédé en continu, par exemple en faisant réagir un mélange de l'alcool benzylique et d'alcanol sur un lit fixe de catalyseur, ces rapports catalyseur/alcool benzylique n'ont pas de sens et à un instant donné, on peut avoir un excès pondéral de catalyseur par rapport à l'alcool benzylique de départ.

La quantité d'alcanol de formule (II) exprimée en moles d'alcanol par mole d'alcool benzylique de formule (I) peut, elle aussi, varier dans de larges limites. Le rapport molaire alcanol de formule (II)/alcool benzylique de formule (I) peut varier entre 1 et 30. La borne supérieure ne présente aucun caractère critique mais toutefois pour des raisons économiques, il n'y a aucun intérêt à la dépasser.

La température de la réaction d'éthérification peut varier largement. Elle est choisie, avantageusement entre 50°C et 200°C et encore plus préférentiellement entre 50°C et 100°C.

Généralement, la réaction est conduite à pression atmosphérique mais des pressions plus élevées peuvent également convenir allant de 1 à 50 bar, de préférence, de 1 à 25 bar. On travaille sous pression autogène lorsque la température de réaction est supérieure à la température d'ébullition des réactifs et/ou des produits.

On préfère conduire la réaction sous atmosphère contrôlée de gaz inertes tels que l'azote ou les gaz rares, par exemple l'argon.

La durée de la réaction peut être très variable. Elle se situe, le plus souvent, entre 15 minutes et 10 heures, de préférence entre 30 minutes et 5 heures.

D'un point de vue pratique, le procédé peut être mis en oeuvre en discontinu ou en continu.

Selon la première variante, on peut charger le catalyseur, l'alcanol de formule (II), éventuellement un solvant organique puis l'on introduit l'alcool benzylique. Un mode préféré de l'invention, consiste à introduire progressivement l'alcool benzylique, en continu ou par fractions puis l'on porte le mélange réactionnel à la température souhaitée.

L'autre variante de l'invention consiste à conduire la réaction en continu, dans un réacteur tubulaire comportant le catalyseur solide disposé en lit fixe.

L'alcool benzylique et l'alcanol sont introduits de préférence, séparément.

Ils peuvent également être introduits dans un solvant tel que mentionné précédemment.

Le temps de séjour du flux de matière sur le lit catalytique varie, par exemple, entre 15 min et 10 heures, et de préférence, entre 30 min et 5 heures.

En fin de réaction, on récupère une phase liquide comprenant l'alcool benzylique éthérifié qui peut être récupéré de manière classique.

Le procédé de l'invention conduit à des alcools de type benzylique éthérifiés répondant préférentiellement à la formule (III) dans laquelle :

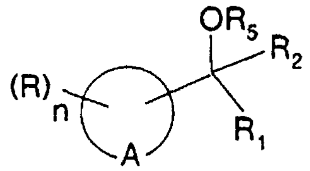

(III)

- A,n, $R_1$, $R_2$ et $R_5$ ont la signification donnée précédemment.

Les composés préférés de l'invention répondent plus particulièrement à la formule (IIIa) dans laquelle :

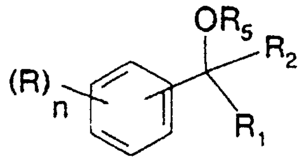

(IIIa)

- n, R, $R_1$, $R_2$ et $R_5$ ont la signification donnée précédemment.

Le procédé de l'invention est particulièrement bien adapté pour la préparation des éthers d'alkyle ($C_1$-$C_4$), de cycloalkyle de l'alcool vanillique.

Un autre objet de la présente invention a pour objet des compositions parfumantes, produits et substances parfumantes caractérisés par le fait qu'ils comprennent à titre de principe actif ayant une influence sur l'odeur, une quantité efficace d'au moins un alcool de type benzylique éthérifié (III) ou (IIIa).

Ainsi, les différents éthers obtenus présentent des propriétés olfactives intéressantes et peuvent être employés, entre autres, pour la préparation de compositions parfumantes et de produits parfumés.

Le méthyl 4-hydroxy-3-méthoxybenzyl éther et le cyclohexyl 4-hydroxy-3-méthoxybenzyl éther se distinguent par leur odeur respectivement de praline pour le premier et de cuir pour le dernier.

Par "compositions parfumantes", on désigne des mélanges de divers ingrédients tels que solvants, supports solides ou liquides, fixateurs, composés odorants divers, etc..., dans lesquels est incorporé l'alcool de type benzylique éthérifié (III) ou (IIIa), lesquels mélanges sont utilisés pour procurer à divers types de produits finis, la fragrance recherchée.

Les bases pour parfum constituent des exemples préférés de compositions parfumantes dans lesquelles l'alcool de type benzylique éthérifié (III) ou (IIIa) peut être avantageusement utilisé.

Les eaux de toilettes, les lotions après rasage, les parfums, les savons, les gels de bain ou de douche ou les produits déodorants ou antiperspirants, qu'ils soient sous forme de sticks ou de lotions constituent des exemples de substances ou de produits finis dans lesquels l'alcool de type benzylique éthérifié (III) ou (IIIa) apporte sa note originale.

Ils peuvent intervenir aussi dans les shampooings et dans les produits capillaires de tout type.

Ils peuvent aussi parfumer les talcs ou poudres de toute nature.

Ils peuvent également convenir pour les désodorisants d'air ambiant ou tout produit d'entretien.

Un autre exemple de compositions dans lesquelles lesdits composés peuvent être introduits de façon avantageuse, est représenté par les compositions détergentes usuelles. Ces compositions comprennent généralement un ou plusieurs des ingrédients suivants : agents tensio-actifs anioniques, cationiques ou amphotères, agents de blanchiment, azurants optiques, charges diverses, agents anti-redéposition. La nature de ces divers composants n'est pas critique et l'alcool de type benzylique éthérifié (III) ou (IIIa) peut être ajouté à tout type de composition détergente. Elles peuvent être introduites dans les adoucissants pour textiles, sous forme liquide ou dans les compositions déposées sur support, le plus souvent un non-tissé, destinées à être employées dans les sèche-linges.

La teneur des compositions selon l'invention en alcool de type benzylique éthérifié (III) ou (IIIa) exprimée en pourcentage en poids dans la composition considérée dépend de la nature de ladite composition (base pour parfum ou eau de toilette par exemple) et de la puissance et de la nature de l'effet recherché au niveau produit final. Il va de soi que dans une base pour parfum la teneur en alcool de type benzylique éthérifié (III) ou (IIIa) peut varier largement, par exemple supérieure à 5 % en poids et peut atteindre 90 % en poids tandis que dans un parfum, une eau de toilette ou une lotion après rasage, cette teneur pourra être très inférieure à 50 % en poids.

La teneur en alcool benzylique éthérifié peut être dans les compositions détergentes, notamment ménagères ou dans des savons, de l'ordre de 0,01 à 2%.

Ils peuvent également intervenir dans les shampooings parfumés à raison de 0,005 à 2 % ou pour parfumer tout produit capillaire.

Ainsi la limite inférieure de la teneur en alcool de type benzylique éthérifié (III) ou (IIIa) peut être celle qui provoque une modification perceptible à l'odorat de la fragrance ou de la note du produit fini. Dans certains cas, cette teneur minimale peut être de l'ordre de 0,001 % en poids. On peut évidemment faire appel à des teneurs non comprises dans les limites des teneurs indiquées ci-avant sans pour autant sortir du cadre de la présente invention.

On donne ci-après des exemples de réalisation de l'invention.

Les exemples qui suivent, illustrent l'invention sans toutefois la limiter.

Par conversion et sélectivité, on entend :

$$\text{Taux de conversion (TT)} = \frac{\text{nombre de moles d'alcool benzylique transformées}}{\text{nombre de moles d'alcool benzylique introduites}} (\%)$$

$$\text{Rendement (RR)} = \frac{\text{nombre de moles d'éther hydroxyalkylé formées}}{\text{nombre de moles d'alcool benzylique introduites}} (\%)$$

<u>Exemples 1 à 5</u>

1 - Dans un réacteur tricol de 100 ml, on introduit 5 g d'alcool vanillique (alcool 4-hydroxy-3-méthoxybenzylique) et 25 g d'alcool aliphatique ROH.

On met en oeuvre un catalyseur comprenant 40 % de liant (alumine) et 60 % d'une zéolithe β commercialisée par la Société PQ. La zéolithe utilisée est une zéolithe de rapport "Si/Al" de 12,5.

On ajoute 2 g de ladite zéolithe calcinée à 500°C.

On agite et on chauffe à 80°C.

On maintient ces conditions pendant 2 heures.

On filtre le catalyseur.

On dose les produits obtenus par chromatographie en phase gazeuse.

On sépare les produits par distillation.

Le produit obtenu selon la réaction suivante est précisée dans le tableau (I) :

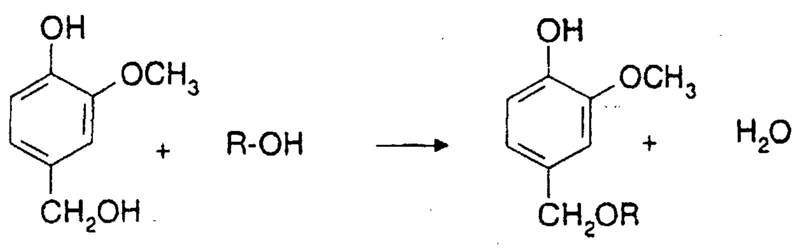

Tableau (I)

| Exemples | R | TT % | RR % |
|---|---|---|---|
| 1 | $CH_3$ | 99 | 95 |
| 2 | $CH_3CH_2$ | 99 | 97 |
| 3 | $CH_3CH_2CH_2$ | 99 | 97 |
| 4 | $CH(CH_3)_2$ | 99 | 95 |
| 5 | $C(CH_3)_3$ | 55 | 37 |

2 - L'odeur du produit de l'exemple 2, à savoir le méthyl 4-hydroxy-3-méthoxybenzyl éther est une odeur de praline tandis que ceux des exemples 3 et 4 ont une odeur légèrement vanillée.

Exemple 6

1 - On reproduit l'exemple 1 à la différence près que l'on met en oeuvre le cyclohexanol.
Les résultats obtenus sont les suivants :

- TT = 99 %
- RR = 96 %

2 - L'odeur du cyclohexyl 4-hydroxy-3-méthoxybenzyl éther représenté par la formule suivante est une odeur cuirée.

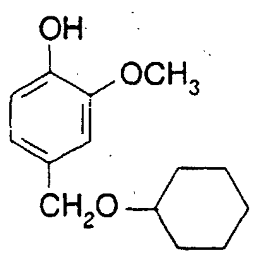

Exemples 7 à 9

1 - On répète le mode opératoire de l'exemple 1.

$$Ar\text{-}CH_2OH + CH_3OH \rightarrow Ar\text{-}CH_2\text{-}O\text{-}CH_3 + H_2O$$

Les résultats obtenus sont consignés dans le tableau (II).

Tableau (II)

| Exemples | Ar | TT % | RR % |
|---|---|---|---|
| 7 | 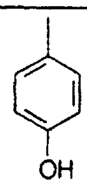 | 99 | 85 |
| 8 | 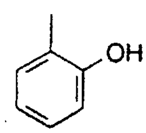 | 99 | 97 |
| 9 | 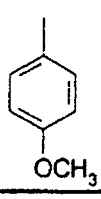 | 99 | 94 |

2 - Les produits des exemples 8 et 9 ont respectivement une odeur animal naphtaline et anisé fromage.

Exemples 10 et 11

On reproduit l'exemple 1 mais en mettant en oeuvre un alcool secondaire.

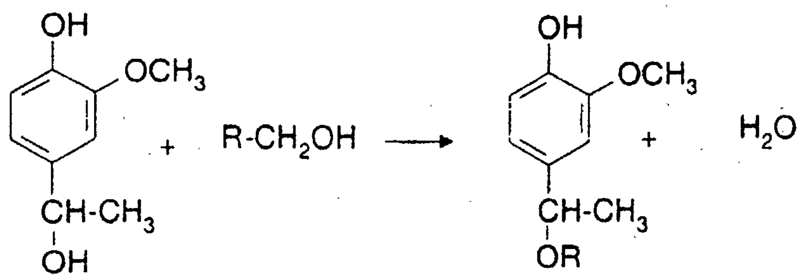

Les résultats obtenus sont rassemblés dans le tableau (III).

Tableau (III)

| Exemples | R | TT % | RR % |
|---|---|---|---|
| 10 | $CH_3$ | 99 | 95 |
| 11 | $CH_3CH_2$ | 99 | 96 |

Exemples 12 et 13

On reproduit l'exemple 1 mais en mettant en oeuvre un alcool insaturé.
Les résultats obtenus sont rassemblés dans le tableau (IV).

Tableau (IV)

| Exemples | R | TT % | RR % |
|---|---|---|---|
| 12 | 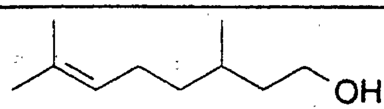 | 100 | 85 |
| 13 | 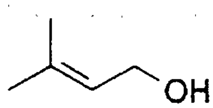 | 100 | 95 |

Exemples 14 et 15

On reproduit l'exemple 1 mais en mettant en oeuvre un alcool secondaire.

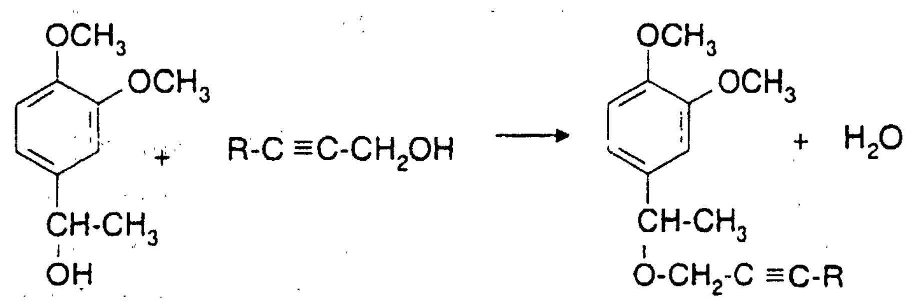

Les résultats obtenus sont rassemblés dans le tableau (V).

Tableau (V)

| Exemples | R | TT % | RR % |
|---|---|---|---|
| 14 | R=H | 100 | 85 |
| 15 | R = $CH_3$ | 100 | 90 |

## Revendications

1. Procédé d'éthérifcation d'un alcool de type benzylique qui consiste à faire réagir ledit alcool avec un autre alcool, en présence d'un catalyseur, ledit procédé étant **caractérisé par le fait que** l'on conduit la réaction d'éthérification en présence d'une quantité efficace d'une zéolithe sous forme acide.

2. Procédé selon la revendication 1 **caractérisé par le fait que** l'alcool benzylique répond à la formule générale (I) :

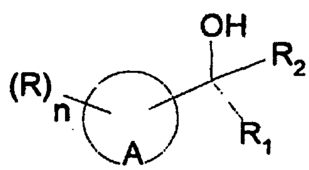

(I)

dans laquelle :

- A symbolise le reste d'un cycle formant tout ou partie d'un système carbocyclique ou hétérocyclique, aromatique, monocyclique ou polycyclique, système comprenant au moins un groupe

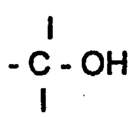

- R représente un ou plusieurs substituants, identiques ou différents,
- $R_1$ et $R_2$, identiques ou différents, représentent un atome d'hydrogène, un groupe fonctionnel ou un groupe hydrocarboné ayant de 1 à 24 atomes de carbone, qui peut être un groupe aliphatique acyclique saturé ou insaturé, linéaire

ou ramifié ; un groupe cycloaliphatique saturé, insaturé ou aromatique, monocyclique ou polycyclique ; un groupe aliphatique saturé ou insaturé, linéaire ou ramifié, porteur d'un substituant cyclique,

- $R_1$ et $R_2$ peuvent former un cycle comprenant éventuellement un autre hétéroatome,
- n est un nombre inférieur ou égal à 5.

3. Procédé selon l'une des revendications 1 et 2 **caractérisé par le fait que** l'alcool de type benzylique répond à la formule (I) dans laquelle $R_1$ et $R_2$, identiques ou différents représentent :

- un groupe aliphatique acyclique, saturé ou insaturé, linéaire ou ramifié, de préférence un groupe alkyle linéaire ou ramifié ayant de 1 à 12 atomes de carbone, de préférence de 1 à 6 atomes de carbone : la chaîne hydrocarbonée pouvant être éventuellement interrompue par un hétéroatome, un groupe fonctionnel et/ou porteuse de substituants,
- un groupe aliphatique acyclique, saturé ou insaturé, linéaire ou ramifié porteur d'un substituant cyclique éventuellement substitué : ledit groupe acyclique pouvant être relié au cycle par un lien valentiel, un hétéroatome ou un groupe fonctionnel,
- un groupe carbocyclique saturé ou comprenant 1 ou 2 insaturations dans le cycle, ayant généralement de 3 à 8 atomes de carbone, de préférence, 6 atomes de carbone dans le cycle ; ledit cycle pouvant être substitué,
- un groupe carbocyclique aromatique, de préférence monocyclique ayant généralement au moins 4 atomes de carbone, de préférence, 6 atomes de carbone dans le cycle ; ledit cycle pouvant être substitué.

et l'un des groupes $R_1$ ou $R_2$ peut représenter un groupe $CF_3$.

4. Procédé selon l'une des revendications 1 à 3 **caractérisé par le fait que** l'alcool de type benzylique répond à la formule générale (I) dans laquelle le reste A est le reste d'un composé cyclique, ayant de préférence, au moins 4 atomes dans le cycle, de préférence, 5 ou 6, éventuellement substitué, et représentant au moins l'un des cycles suivants :

- un carbocycle aromatique, monocyclique ou polycyclique, de préférence, un cycle benzénique ou naphtalénique,
- un hétérocycle aromatique, monocyclique ou polycyclique comportant au moins un des hétéroatomes O, N et S,

5. Procédé selon la revendication 4 **caractérisé par le fait que** l'alcool de type benzylique répond à la formule générale (I) dans laquelle le reste A peut être porteur d'un ou plusieurs groupes électro-donneurs tels que :

- les groupes alkyle linéaires ou ramifiés ayant de préférence de 1 à 6 atomes de carbone et encore plus préférentiellement de 1 à 4 atomes de carbone,
- les groupes alcényle linéaires ou ramifiés ayant de préférence de 2 à 6 atomes de carbone et encore plus préférentiellement de 2 à 4 atomes de carbone,
- les groupes halogénoalkyle linéaires ou ramifiés ayant de préférence de 1 à 6 atomes de carbone et encore plus préférentiellement de 1 à 4 atomes de carbone,
- les groupes cycloalkyle ayant de 3 à 6 atomes de carbone, de préférence, le groupe cyclohexyle,
- le groupe phényle,
- le groupe hydroxyle,
- le groupe $NO_2$,
- les groupes alkoxy $R_3$-O- ou thioéther $R_3$-S- dans lesquels $R_3$ représente un groupe alkyle linéaire ou ramifié ayant de 1 à 6 atomes de carbone et encore plus préférentiellement de 1 à 4 atomes de carbone ou le groupe phényle,
- les groupes -N-$(R_4)_2$ dans lesquels les groupes $R_4$ identiques ou différents représentent un atome d'hydrogène, un groupe alkyle linéaire ou ramifié ayant de 1 à 6 atomes de carbone et encore plus préférentiellement de 1 à 4 atomes de carbone ou un groupe phényle,
- les groupes -NH-CO-$R_4$ dans lesquels le groupe $R_4$ a la signification donnée précédemment,
- les groupes carboxy ou dérivé $R_4$-O-CO- dans lesquels le groupe $R_4$ a la signification donnée précédemment,
- les groupes acyloxy ou aroyloxy $R_3$-CO-O- dans lesquels le groupe $R_3$ a la signification donnée précédemment,
- un atome d'halogène, de préférence, un atome de fluor,
- un groupe $CF_3$.

6. Procédé selon l'une des revendications 1 à 5 **caractérisé par le fait que** l'alcool de type benzylique répond à la formule générale (I) dans laquelle lorsque n est supérieur ou égal à 2, deux groupes R et les 2 atomes successifs du cycle aromatique peuvent être liés entre eux par un groupe alkylène, alcénylène ou alcénylidène ayant de 2 à 4 atomes de carbone pour former un hétérocycle saturé, insaturé ou aromatique ayant de 5 à 7 atomes de carbone : un ou plusieurs atomes de carbone pouvant être remplacés par un autre hétéroatome, de préférence l'oxygène.

**7.** Procédé selon l'une des revendications 1 à 6 **caractérisé par le fait que** l'alcool de type benzylique répond à la formule (Ia) :

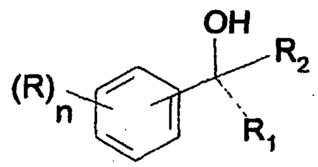

(Ia)

dans laquelle :

- n est un nombre inférieur ou égal à 4, de préférence égal à 0, 1 ou 2,
- le groupe R est un groupe électro-donneur, de préférence, un groupe alkyle, alkoxy ou méthytènedioxy ou éthylènedioxy,
- les groupes $R_1$ et $R_2$, identiques ou différents, représentent :
  - . un atome d'hydrogène,
  - . un groupe alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  - . un groupe cycloalkyle ayant de 3 à 8 atomes de carbone, de préférence, un groupe cyclopentyle ou cyclohexyle,
  - . un groupe phényle,
  - . un groupe phénylalkyle ayant de 7 à 12 atomes de carbone, de préférence, un groupe benzyle,
  - . un groupe $CF_3$.

**8.** Procédé selon l'une des revendications 1 à 7 **caractérisé par le fait que** l'alcool de type benzylique est choisi parmi :

- l'alcool vanillique,
- l'alcool p-hydroxybenzylique,
- le 1-(4-hydroxy-3-méthoxyphényl)éthanol,
- l'alcool 2-hydroxybenzylique,
- l'alcool p-méthoxybenzylique,
- l'alcool 3,4-diméthoxybenzylique,
- l'alcool 6-n-propyl-3,4-diméthoxybenzylique,
- l'alcool (3,4-diméthoxyphényl)diméthylcarbinol,
- le 1-[1-hydroxy-2-méthylpropyl]-3,4-diméthoxybenzène,
- le 1-[1-hydroxy-2-méthylpropyl]-3,4-diéthoxybenzène,
- le 1-[1-hydroxyéthyl]-3,4-diéthoxybenzène,
- le 1-[1-hydroxyéthyl]-3,4-diméthoxy-6-propylbenzène,
- le 5-[1-hydroxyéthyl]-1,3-benzodioxol,
- le naphtalène-2-méthylol.

**9.** Procédé selon l'une des revendications 1 à 8 **caractérisé par le fait que** l'alcanol mis en oeuvre répond à la formule générale (II) :

$$R_5 - OH \qquad (II)$$

dans ladite formule (II) :

- $R_5$ représente un groupe hydrocarboné ayant de 1 à 24 atomes de carbone, qui peut être un groupe aliphatique acyclique saturé ou insaturé, linéaire ou ramifié ; un groupe cycloaliphatique saturé, insaturé ou aromatique ; un groupe aliphatique saturé ou insaturé, linéaire ou ramifié, porteur d'un substituant cyclique.

**10.** Procédé selon la revendication 9 **caractérisé par le fait que** l'alcanol répond à la formule (II) dans laquelle $R_5$ représente :

- un groupe aliphatique acyclique, saturé ou insaturé, linéaire ou ramifié, de préférence, un groupe alkyle, alcényle, alcadiényle, alcynyle, linéaire ou ramifié ayant de préférence de 1 à 24 atomes de carbone : la chaîne hydrocarbonée pouvant être éventuellement interrompue par un groupe fonctionnel et/ou porteuse de substituants
- un groupe aliphatique acyclique, saturé ou insaturé, linéaire ou ramifié porteur. d'un substituant cyclique éventuellement substitué : ledit groupe acyclique pouvant être relié au cycle par un lien valentiel, un hétéroatome ou un groupe fonctionnel,
- un groupe carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué.

**11.** Procédé selon l'une des revendications 9 et 10 **caractérisé par le fait que** l'alcanol est un alcool terpénique de formule (IIa) :

$$T - OH \quad (IIa)$$

dans ladite formule (IIa) :

- T représente le reste d'un alcool terpénique ayant un nombre d'atomes de carbone multiple de 5.

**12.** Procédé selon la revendication 11 **caractérisé par le fait que** l'alcool terpénique mis en oeuvre répond à la formule générale (IIa) dans laquelle le reste T représente un groupe hydrocarboné ayant de 5 à 40 atomes de carbone et plus particulièrement un groupe aliphatique, saturé ou insaturé, linéaire ou ramifié ; un groupe cycloaliphatique, saturé, insaturé ou aromatique, monocyclique ou polycyclique, comprenant des cycles ayant de 3 à 8 atomes de carbone.

**13.** Procédé selon l'une des revendications 11 et 12 **caractérisé par le fait que** le reste T représente le reste :

- d'un alcool terpénique aliphatique, saturé ou insaturé, linéaire ou ramifié,
- d'un alcool terpénique cycloaliphatique, monocyclique, saturé ou insaturé, ou aromatique,
- d'un alcool terpénique cycloaliphatique, polycyclique comprenant au moins deux carbocycles, saturés et/ou insaturés.

**14.** Procédé selon la revendication 13 **caractérisé par le fait que** l'alcool de formule (II) est choisi parmi :

. le méthanol,
. l'éthanol,
. le trifluoroéthanol,
. le propanol, l'alcool isopropylique,
. le butanol, l'alcool isobutylique, l'alcool sec-butylique, l'alcool tert-butylique,
. le pentanol, l'alcool isopentylique, l'alcool sec-pentylique et l'alcool tert-pentylique,
. le 3-chlorobut-2-én-1-ol,
. le 2-butyn-1-ol,
. le 3,7-diméthyloct-6-én-1-ol,
. l'alcool chrysantémique,
. le 3,7-diméthyloctanol,
. le géraniol,
. le linalool,
. le citronellol,
. l'hydoxycitronellol,
. le nérol,
. le thymol,
. le menthol,
. l'isoboméol,
. le verbénol.

**15.** Procédé selon l'une des revendications 1 à 14 **caractérisé par le fait que** le catalyseur est une zéolithe naturelle ou synthétique.

**16.** Procédé selon la revendication 15 **caractérisé par le fait que** la zéolithe est choisie parmi :

- les zéolithes naturelles telles que la chabazite, la clinoptilolite, l'érionite, la mordénite, la phillipsite, l'offrétite.
- les zéolithes synthétiques à réseau monodimensionnel telles que la zéolithe ZSM-4, la zéolithe L, la zéolithe ZSM-12, la zéolithe ZSM-22, la zéolithe ZSM-23, la zéolithe ZSM-48,
- les zéolithes à réseau bidimensionnel telles que la mordénite, la ferrierite,
- les zéolithes à réseau tridimensionnel telles que la zéolithe β, la zéolithe Y, la zéolithe X, la zéolithe ZSM-5, la zéolithe ZSM-11, l'offrétite,
- la zéolithe mésoporeuse de type MCM.

**17.** Procédé selon la revendication 16 **caractérisé par le fait que** la zéolithe est une zéolithe β.

**18.** Procédé selon la revendication 17 **caractérisé par le fait que** la zéolithe β a un rapport molaire Si/Al supérieur à 8, de préférence entre 10 à 100, et encore plus préférentiellement de 12 à 50.

**19.** Procédé selon l'uns des revendications 15 à 18 **caractérisé par le fait que** la zéolithe est mise en oeuvre seule ou en mélange avec une matrice minérale choisie, de préférence, parmi les oxydes de métaux, tels que les oxydes d'aluminium, de silicium et/ou de zirconium, ou encore parmi les argiles et plus particulièrement, le kaolin, le talc ou la montmorillonite.

**20.** Procédé selon l'une des revendications 1 à 19 **caractérisé par le fait que** le rapport entre le nombre de moles d'alcanol et le nombre de moles d'alcool de type benzylique varie entre 1 et 30.

**21.** Procédé selon l'une des revendications 1 à 20 **caractérisé par le fait que** la quantité de catalyseur représente en poids par rapport au réactif en défaut, de 2 à 50 %, de préférence, de 5 à 20 %.

**22.** Procédé selon l'une des revendications 1 à 21 **caractérisé par le fait que** la température à laquelle est mise en oeuvre la réaction d'éthérification se situe entre 50°C et 200°C, de préférence entre 50°C et 100°C.

**23.** Procédé selon l'une des revendications 1 à 22 **caractérisé par le fait que** la réaction est conduite sous pression atmosphérique.

**24.** Procédé selon l'une des revendications 1 à 23 **caractérisé par le fait que** le temps de séjour du flux de matière sur le lit catalytique varie entre 15 min et 10 h, et de préférence, entre 30 min et 5 h.

**25.** Procédé selon l'une des revendications 1 à 24 **caractérisé par le fait que** l'on obtient en fin de réaction, une phase liquide comprenant l'alcool de type benzylique éthérifié qui peut être récupéré de manière classique.

**26.** Cyclohexyl 4-hydroxy-3-méthoxybenzyl éther.

**27.** Utilisation du cyclohexyl 4-hydroxy-3-méthoxybenzyl éther, comme ingrédient parfumant dans le domaine de la parfumerie.

**28.** Procédé pour l'obtention de compositions parfumantes, de substances et produits parfumés destinés à la parfumerie **caractérisé par le fait que** l'on ajoute aux constituants usuels de ces compositions, substances et produits finis, une quantité efficace de cyclohexyl 4-hydroxy-3-méthoxybenzyl éther.

**29.** Compositions parfumantes, substances et produits parfumés **caractérisés par le fait qu'**ils comprennent, à titre de principe actif ayant une influence sur l'odeur, une quantité efficace de cyclohexyl 4-hydroxy-3-méthoxybenzyl éther.

**30.** Article parfumé selon la revendication 29 sous forme de parfum, d'eau de toilette, de lotions après rasage, de parfums, de savons, de gels de bain ou de douche, de produits déodorants ou antiperspirants, de shampooings ou tout produit capillaire, de talcs ou poudres de toute nature, de désodorisants d'air ambiant, de tout produit d'entretien ou de compositions détergentes, d'adoucissants pour textiles.

## Claims

1. A process for etherification of a benzyl type alcohol, consisting of reacting said alcohol with a further alcohol in the presence of a catalyst, said process being **characterized in that** the etherification reaction is carried out in the presence of an effective quantity of a zeolite in its acid form.

2. A process according to claim 1, **characterized in that** the benzyl alcohol has general formula (I):

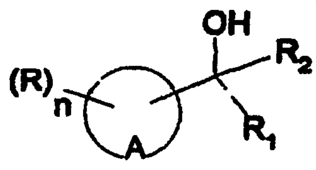

(I)

in which:

- A represents the residue of a cycle forming all or a portion of a monocyclic or polycyclic, aromatic carbocyclic or heterocyclic system comprising at least one group

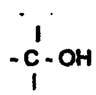

- R represents one or more substituents which can be identical or different;
- $R_1$ and $R_2$, which may be identical or different, represent a hydrogen atom, a functional group or a hydrocarbon group containing 1 to 24 carbon atoms, which may be a linear or branched, saturated or unsaturated acyclic aliphatic group; a monocyclic or polycyclic, saturated, unsaturated or aromatic cycloaliphatic group; or a linear or branched, saturated or unsaturated aliphatic group carrying a cyclic substituent;
- $R_1$ and $R_2$ may form a cycle optionally comprising a further heteroatom;
- n is a number that is 5 or less.

3. A process according to claim 1 or claim 2, **characterized in that** the benzyl type alcohol has formula (I) in which $R_1$ and $R_2$, which may be identical or different, represent:

- a linear or branched, saturated or unsaturated acyclic aliphatic group, preferably a linear or branched alkyl group containing 1 to 12 carbon atoms, more preferably 1 to 6 carbon atoms: the hydrocarbon chain can optionally be interrupted by a heteroatom, a functional group and/or it may carry substituents;
- a linear or branched, saturated or unsaturated acyclic aliphatic group carrying a cyclic substituent which may be substituted: said acyclic group may be connected to the cycle via a covalent bond, a heteroatom or a functional group;
- a carbocyclic group that is saturated or comprises 1 or 2 unsaturated bonds in the cycle, generally containing 3 to 8 carbon atoms, preferably 6 carbon atoms in the cycle; said cycle may be substituted;
- an aromatic carbocyclic group, preferably monocyclic generally containing at least 4 carbon atoms, preferably 6 carbon atoms in the cycle; said cycle may be substituted;

and one of groups $R_1$ or $R_2$ can represent a $CF_3$ group.

4. A process according to one of claims 1 to 3, **characterized in that** the benzyl type alcohol has general formula (I) in which residue A is the residue of a cyclic compound, preferably containing at least 4 atoms in the cycle, more preferably 5 or 6, which may be substituted, and representing at least one of the following cycles:

- a monocyclic or polycyclic aromatic carbocycle, preferably a benzene or naphthalene cycle;
- a monocyclic or polycyclic aromatic heterocycle comprising at least one of heteroatoms O, N and S.

**5.** A process according to claim 4, **characterized in that** the benzyl type alcohol has general formula (I) in which residue A can carry one or more electron-donating groups such as:

- linear or branched alkyl groups preferably containing 1 to 6 carbon atoms, more preferably containing 1 to 4 carbon atoms;
- linear or branched alkenyl groups preferably containing 2 to 6 carbon atoms, more preferably containing 2 to 4 carbon atoms;
- linear or branched halogenoalkyl groups preferably containing 1 to 6 carbon atoms, more preferably 1 to 4 carbon atoms;
- cycloalkyl groups containing 3 to 6 carbon atoms, preferably the cyclohexyl group;
- the phenyl group;
- the hydroxyl group;
- the $NO_2$ group;
- alkoxy $R_3$-O- or thioether $R_3$-S- groups, in which $R_3$ represents a linear or branched alkyl group containing 1 to 6 carbon atoms, more preferably 1 to 4 carbon atoms or the phenyl group;
- -N-$(R_4)_2$ groups in which group $R_4$, which may be identical or different, represents a hydrogen atom, a linear or branched alkyl group containing 1 to 6 carbon atoms, more preferably 1 to 4 carbon atoms, or a phenyl group;
- a -NH-CO-$R_4$ group, in which $R_4$ has the meaning given above;
- carboxy groups or $R_4$-O-CO- derivatives, in which group $R_4$ has the meaning given above;
- acyloxy or aroyloxy groups $R_3$-CO-O-, in which group $R_3$ has the meaning given above;
- a halogen atom, preferably a fluorine atom;
- a $CF_3$ group.

**6.** A process according to one of claims 1 to 5, **characterized in that** the benzyl type alcohol has general formula (I) in which when n is 2 or more, two groups R and the next 2 atoms of the aromatic cycle can be bonded together via an alkylene, alkenylene or alkenylidene group containing 2 to 4 carbon atoms to form a saturated, unsaturated or aromatic heterocycle containing 5 to 7 carbon atoms: one or more carbon atoms can be replaced by a further heteroatom, preferably oxygen.

**7.** A process according to one of claims 1 to 6, **characterized in that** the benzyl type alcohol has formula (Ia):

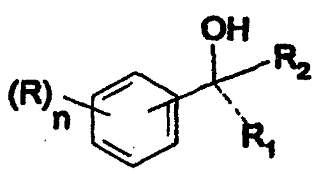

(Ia)

in which:

- n is a number that is 4 or less, preferably 0, 1 or 2;
- group R is an electron donor, preferably an alkyl, alkoxy, methylenedioxy or ethylenedioxy group;
- groups $R_1$ and $R_2$, which may be identical or different, represent:

  o a hydrogen atom;
  o a linear or branched alkyl group containing 1 to 6 carbon atoms, preferably 1 to 4 carbon atoms, such as methyl, ethyl, propyl, isopropyl, butyl, isobutyl, sec-butyl or tert-butyl;
  o a cycloalkyl group containing 3 to 8 carbon atoms, preferably a cyclopentyl or cyclohexyl group;
  o a phenyl group;
  o a phenylalkyl group containing 7 to 12 carbon atoms, preferably a benzyl group;
  o a $CF_3$ group.

**8.** A process according to one of claims 1 to 7, **characterized in that** the benzyl type alcohol is selected from:

- vanillic alcohol;
- p-hydroxybenzyl alcohol;

- 1-(4-hydroxy-3-methoxyphenyl)ethanol;
- 2-hydroxybenzyl alcohol;
- p-methoxybenzyl alcohol;
- 3,4-dimethoxybenzyl alcohol;
- 6-n-propyl-3,4-dimethoxybenzyl alcohol;
- (3,4-dimethoxyphenyl)dimethylcarbinol;
- 1-[1-hydroxy-2-methylpropyl]-3,4-dimethoxybenzene;
- 1-[1-hydroxy-2-methylpropyl]-3,4-diethoxybenzene;
- 1-[1-hydroxyethyl]-3,4-diethoxybenzene;
- 1-[1-hydroxyethyl]-3,4-dimethoxy-6-propylbenzene;
- 5-[1-hydroxyethyl]-1,3-benzodioxol;
- naphthalene-2-methylol.

**9.** A process according to one of claims 1 to 8, **characterized in that** the alkanol used has general formula (II):

$$R_5\text{-OH} \qquad (II)$$

in which formula (II):

- $R_5$ represents a hydrocarbon group containing 1 to 24 carbon atoms, which may be a linear or branched, saturated or unsaturated acyclic aliphatic group; a saturated, unsaturated or aromatic cycloaliphatic group; or a linear or branched, saturated or unsaturated aliphatic group carrying a cyclic substituent.

**10.** A process according to claim 9, **characterized in that** the alkanol has formula (II) in which $R_5$ represents:

- a linear or branched, saturated or unsaturated acyclic aliphatic group, preferably a linear or branched alkyl, alkenyl, alkadienyl or alkynyl group preferably containing 1 to 24 carbon atoms: the hydrocarbon chain may optionally be interrupted by a functional group and/or may carry substituents;
- a linear or branched, saturated or unsaturated acyclic aliphatic group carrying a cyclic substituent that may optionally be substituted: said acyclic group can be bonded to the cycle via a covalent bond, a heteroatom or a functional group;
- a carbocyclic group that is saturated or comprises 1 or 2 unsaturated bonds in the cycle, generally containing 3 to 7 carbon atoms, preferably 6 carbon atoms in the cycle; said cycle may be substituted.

**11.** A process according to one of claims 9 or 10, **characterized in that** the alkanol is a terpene alcohol with formula (IIa):

$$T\text{-OH} \qquad (IIa)$$

in which formula (IIa):

- T represents the residue of a terpene alcohol containing a number of carbon atoms that is a multiple of 5.

**12.** A process according to claim 11, **characterized in that** the terpene alcohol employed has general formula (IIa) in which residue T represents a hydrocarbon group containing 5 to 40 carbon atoms, more particularly a linear or branched, saturated or unsaturated aliphatic group; or a monocyclic or polycyclic, saturated, unsaturated or aromatic cycloaliphatic group comprising cycles containing 3 to 8 carbon atoms.

**13.** A process according to claim 11 or claim 12, **characterized in that** residue T represents the residue:

- of a linear or branched, saturated or unsaturated aliphatic terpene alcohol;
- of a saturated, unsaturated or aromatic monocyclic cycloaliphatic terpene alcohol;
- of a polycyclic cycloaliphatic terpene alcohol comprising at least two saturated and/or unsaturated carbocycles.

**14.** A process according to claim 13, **characterized in that** the alcohol with formula (II) is selected from:

- methanol;
- ethanol;
- trifluoroethanol;
- propanol, isopropyl alcohol;
- butanol, isobutyl alcohol, sec-butyl alcohol, tert-butyl alcohol;
- pentanol, isopentyl alcohol, sec-pentyl alcohol and tert-pentyl alcohol;
- 3-chlorobut-2-en-1-ol;
- 2-butyn-1-ol;
- 3,7-dimethyloct-6-en-1-ol;
- chrysanthemic alcohol;
- 3,7-dimethyloctanol;
- gerianol;
- linalool;
- citronellol;
- hydroxycitronellol;
- nerol;
- thymol;
- menthol;
- isoborneol;
- verbenol.

**15.** A process according to one of claims 1 to 14, **characterized in that** the catalyst is a natural or synthetic zeolite.

**16.** A process according to claim 15, **characterized in that** the zeolite is selected from:

- natural zeolites such as chabazite, clinoptilolite, erionite, mordenite, phillipsite or offretite;
- synthetic zeolites with a monodimensional lattice such as ZSM-4 zeolite, L zeolite, ZSM-12 zeolite, ZSM-22 zeolite, ZSM-23 zeolite or ZSM-48 zeolite;
- zeolites with a two-dimensional lattice such as mordenite or ferrierite;
- zeolites with a three-dimensional lattice such as β zeolite, Y zeolite, X zeolite, ZSM-5 zeolite, ZSM-11 zeolite or offretite;
- mesoporous MCM type zeolite.

**17.** A process according to claim 16, **characterized in that** the zeolite is a β zeolite.

**18.** A process according to claim 17, **characterized in that** the β zeolite has a Si/Al mole ratio of more than 8, preferably between 10 and 100, more preferably between 12 and 50.

**19.** A process according to one of claims 15 to 18, **characterized in that** the zeolite is employed alone or as a mixture with a mineral matrix preferably selected from metal oxides, such as aluminium oxide, silicon oxide and/or zirconium oxide, or from clays, more particularly kaolin, talc or montmorillonite.

**20.** A process according to one of claims 1 to 19, **characterized in that** the ratio between the number of moles of alkanol and the number of moles of benzyl type alcohol is in the range 1 to 30.

**21.** A process according to one of claims 1 to 20, **characterized in that** the quantity of catalyst represents, by weight with respect to the minor reagent, 2% to 50%, preferably 5% to 20%.

**22.** A process according to one of claims 1 to 21, **characterized in that** the temperature at which the etherification reaction is carried out is in the range 50°C to 200°C, preferably in the range 50°C to 100°C.

**23.** A process according to one of claims 1 to 22, **characterized in that** the reaction is carried out at atmospheric pressure.

**24.** A process according to one of claims 1 to 23, **characterized in that** the residence time for the stream of material over the catalytic bed is in the range 15 minutes to 10 hours, preferably in the range 30 minutes to 5 hours.

**25.** A process according to one of claims 1 to 24, **characterized in that** at the end of the reaction, a liquid phase is

obtained comprising the etherified benzyl type alcohol which can be recovered conventionally.

**26.** Cyclohexyl-4-hydroxy-3-methoxybenzyl ether.

**27.** Use of cyclohexyl-4-hydroxy-3-methoxybenzyl ether as a perfuming ingredient in the perfume field.

**28.** A process for producing perfuming compositions, perfumed substances and products intended for perfumery, **characterized in that** an effective quantity of cyclohexyl-4-hydroxy-3-methoxybenzyl ether is added to the usual constituents of said compositions, substances and finished products.

**29.** Perfumed compositions, substances and perfumed products, **characterized in that** they comprise an effective quantity of cyclohexyl-4-hydroxy-3-methoxybenzyl ether as the active principle having an influence on the odour.

**30.** A perfumed article according to claim 29 in the form of a perfume, toilet water, after-shave lotion, fragrance, soap, bath or shower gel, deodorant or antiperspirant product, shampoo or any hair product, talc or powder of any nature, air freshener, any cleaning product or detergent composition or textile conditioner.

**Patentansprüche**

**1.** Verfahren zur Veretherung eines Benzylalkohols, bei dem man den Alkohol mit einem anderen Alkohol in Gegenwart eines Katalysators reagieren läßt, **dadurch gekennzeichnet, daß** man die Veretherungsreaktion in Gegenwart einer wirksamen Menge eines Zeoliths in saurer Form durchführt.

**2.** Verfahren nach Anspruch 1, **dadurch gekennzeichnet, daß** der Benzylalkohol der allgemeinen Formel (I) entspricht:

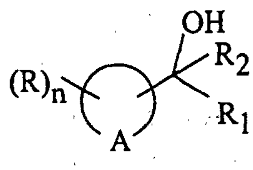

(I)

wobei:

- A den Rest eines Rings darstellt, der ganz oder teilweise ein monocyclisches oder polycyclisches, aromatisches, carbocyclisches oder heterocyclisches System bildet, wobei das System mindestens eine Gruppe

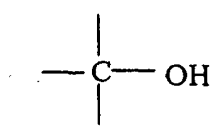

umfaßt;

- R einen oder mehrere Substituenten, identisch oder verschieden, darstellt;

- $R_1$ und $R_2$, identisch oder verschieden, ein Wasserstoffatom, eine funktionelle Gruppe oder eine Kohlenwasserstoffgruppe mit 1 bis 24 Kohlenstoffatomen, die eine lineare oder verzweigte, gesättigte oder ungesättigte acyclische aliphatische Gruppe sein kann, eine monocyclische oder polycyclische, gesättigte, ungesättigte oder aromatische, cycloaliphatische Gruppe oder eine lineare oder verzweigte, gesättigte oder ungesättigte, aliphatische Gruppe, die einen ringförmigen Substituenten trägt, darstellt;

- $R_1$ und $R_2$ einen Ring bilden können, der gegebenenfalls ein weiteres Heteroatom umfaßt;

- n eine ganze Zahl kleiner oder gleich 5 ist.

**3.** Verfahren nach Anspruch 1 oder 2, **dadurch gekennzeichnet, daß** der Benzylalkohol der Formel (I) entspricht, in der $R_1$ und $R_2$, identisch oder verschieden, darstellen:

- eine lineare oder verzweigte, gesättigte oder ungesättigte, acyclische aliphatische Gruppe, vorzugsweise eine lineare oder verzweigte Alkylgruppe mit 1 bis 12 Kohlenstoffatomen, vorzugsweise 1 bis 6 Kohlenstoffatomen, wobei die Kohlenwasserstoffkette gegebenenfalls durch ein Heteroatom oder eine funktionelle Gruppe unterbrochen sein und/oder Träger von Substituenten sein kann;

- eine lineare der verzweigte, gesättigte oder ungesättigte, acyclische aliphatische Gruppe, die einen gegebenenfalls substituierten ringförmigen Substituenten trägt, wobei die acyclische Gruppe über eine Valenzbindung, ein Heteroatom oder eine funktionelle Gruppe mit dem Ring verbunden sein kann;

- eine carbocyclische Gruppe, die gesättigt ist oder ein oder zwei ungesättigte Bindungen im Ring umfaßt, im allgemeinen mit 3 bis 8 Kohlenstoffatomen, vorzugsweise 6 Kohlenstoffatomen im Ring, wobei der Ring substituiert sein kann;

- eine vorzugsweise monocyclische, aromatische carbocyclische Gruppe mit im allgemeinen mindestens 4 Kohlenstoffatomen, vorzugsweise 6 Kohlenstoffatomen im Ring, wobei der Ring substituiert sein kann,

wobei eine der Gruppen $R_1$ oder $R_2$ eine $CF_3$-Gruppe darstellen kann.

**4.** Verfahren nach einem der Ansprüche 1 bis 3, **dadurch gekennzeichnet, daß** der Benzylalkohol der allgemeinen Formel (I) entspricht, wobei der Rest A der Rest einer gegebenenfalls substituierten ringförmigen Verbindung mit vorzugsweise mindestens 4 Atomen im Ring, vorzugsweise 5 oder 6 Atomen, ist, der mindestens einen der folgenden Ringe darstellt:

- einen polycyclischen oder monocyclischen, aromatischen Carbozyklus, vorzugsweise einen Benzol- oder Naphthalinring;

- einen polycyclischen oder monocyclischen, aromatischen Heterozyklus mit mindestens einem der Heteroatome O, N und S.

**5.** Verfahren nach Anspruch 4, **dadurch gekennzeichnet, daß** der Benzylalkohol der allgemeinen Formel (I) entspricht, in welcher der Rest A eine oder mehrere Elektronendonorgruppen trägt, wie:

- lineare oder verzweigte Alkylgruppen mit vorzugsweise 1 bis 6 Kohlenstoffatome, bevorzugt 1 bis 4 Kohlenstoffatomen,

- lineare oder verzweigte Alkenylgruppen mit vorzugsweise 2 bis 6 Kohlenstoffatomen, bevorzugt 2 bis 4 Kohlenstoffatomen,

- lineare oder verzweigte Halogenalkylgruppen mit vorzugsweise 1 bis 6 Kohlenstoffatomen, bevorzugt 1 bis 4 Kohlenstoffatomen,

- Cycloalkylgruppen mit 3 bis 6 Kohlenstoffatomen, vorzugsweise eine Cyclohexylgruppe,

- eine Phenylgruppe,

- eine Hydroxylgruppe,

- eine $NO_2$-Gruppe,

- Alkoxygruppen $R_3$-O- oder Thioethergruppen $R_3$-S-, wobei $R_3$ eine lineare oder verzweigte Alkylgruppe mit 1 bis 6 Kohlenstoffatomen, bevorzugt 1 bis 4 Kohlenstoffatomen, oder eine Phenylgruppe darstellt,

- Gruppen -N-$(R_4)_2$, wobei die Gruppen $R_4$, identisch oder verschieden, ein Wasserstoffatom, einen linearen

oder verzweigten Alkylrest mit 1 bis 6 Kohlenstoffatomen, bevorzugt 1 bis 4 Kohlenstoffatomen, oder eine Phenylgruppe darstellen,

- Gruppen -NH-CO-$R_4$, wobei die Gruppe $R_4$ die zuvor angegebene Bedeutung hat,
- Carboxylgruppen oder hiervon abgeleitete Gruppen $R_4$-O-CO-, wobei die Gruppe $R_4$ die zuvor angegebene Bedeutung hat,
- Acyloxygruppen oder Aryloxygruppen $R_3$-CO-O-, wobei die Gruppe $R_3$ die zuvor angegebene Bedeutung hat,
- ein Halogenatom, vorzugsweise ein Fluoratom,
- eine $CF_3$-Gruppe.

**6.** Verfahren nach einem der Ansprüche 1 bis 5, **dadurch gekennzeichnet, daß** der Benzylalkohol der allgemeinen Formel (I) entspricht, wobei, wenn n größer oder gleich 2 ist, zwei Gruppen R und die 2 aufeinanderfolgenden Atome des aromatischen Rings untereinander über eine Alkylen-, Alkenylen- oder Alkenylidengruppe mit 2 bis 4 Kohlenstoffatomen verbunden sein können, um einen gesättigten, ungesättigten oder aromatischen Heterozyklus mit 5 bis 7 Kohlenstoffatomen zu bilden, wobei eines oder mehrere der Kohlenstoffatome durch ein weiteres Heteroatom, vorzugsweise Sauerstoff, ersetzt sein kann.

**7.** Verfahren nach einem der Ansprüche 1 bis 6, **dadurch gekennzeichnet, daß** der Benzylalkohol der Formel (Ia) entspricht:

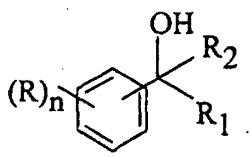
(Ia)

wobei:

- n eine Zahl kleiner oder gleich 4, vorzugsweise gleich 0, 1 oder 2, ist;
- die Gruppe R eine Elektonendonorgruppe, vorzugsweise eine Alkyl-, Alkoxy- oder Methylendioxy- oder Ethylendioxygruppe, ist;
- die Gruppen $R_1$ und $R_2$, identisch oder verschieden, darstellen:
    - · ein Wasserstoffatom,
    - · eine lineare oder verzweigte Alkylgruppe mit 1 bis 6 Kohlenstoffatomen, vorzugsweise 1 bis 4 Kohlenstoffatomen, wie Methyl, Ethyl, Propyl, Isopropyl, Butyl, Isobutyl, sek.-Butyl und tert.-Butyl,
    - · eine Cycloalkylgruppe mit 3 bis 8 Kohlenstoffatomen, vorzugsweise eine Cyclopentyl- oder Cyclohexylgruppe,
    - · eine Phenylgruppe,
    - · eine Phenylalkylgruppe mit 7 bis 12 Kohlenstoffatomen, vorzugsweise eine Benzylgruppe,
    - · eine $CF_3$-Gruppe.

**8.** Verfahren nach einem der Ansprüche 1 bis 7, **dadurch gekennzeichnet, daß** der Benzylalkohol ausgewählt ist aus:

- Vanillinalkohol,
- p-Hydroxybenzylalkohol,
- 1-(4-Hydroxy-3-methoxyphenyl)ethanol,
- 2-Hydroxybenzylalkohol,
- p-Methoxybenzylalkohol,
- 3,4-Dimethoxybenzylalkohol,
- 6-n-Propyl-3,4-dimethoxybenzylalkohol,
- (3,4-Dimethoxyphenyl)dimethylcarbinolalkohol,
- 1-[1-Hydroxy-2-methylpropyl]-3,4-dimethoxybenzol,
- 1-[1-Hydroxy-2-methylpropyl]-3,4-diethoxybenzol,
- 1-[1-Hydroxyethyl]-3,4-diethoxybenzol,
- 1-[1-Hydroxyethyl]-3,4-dimethoxy-6-propylbenzol,
- 5-[1-Hydroxyethyl]-1,3-benzodioxol,
- Naphthalin-2-methylol.

**9.** Verfahren nach einem der Ansprüche 1 bis 8, **dadurch gekennzeichnet, daß** das eingesetzte Alkanol der allgemeinen Formel (II) entspricht:

$$R_5\text{-OH} \qquad (II)$$

wobei in der Formel (II):

- $R_5$ eine Kohlenwasserstoffgruppe mit 1 bis 24 Kohlenstoffatomen darstellt, die eine lineare oder verzweigte, gesättigte oder ungesättigte, acyclische aliphatische Gruppe, eine gesättigte, ungesättigte oder aromatische, cycloaliphatische Gruppe oder eine lineare oder verzweigte, gesättigte oder ungesättigte aliphatische Gruppe, die einen ringförmigen Substituenten trägt, sein kann.

**10.** Verfahren nach Anspruch 9, **dadurch gekennzeichnet, daß** das Alkanol der Formel (II) entspricht, in der $R_5$ darstellt:

- eine lineare oder verzweigte, gesättigte oder ungesättigte, acyclische aliphatische Gruppe, vorzugsweise eine lineare oder verzweigte Alkyl-, Alkenyl-, Alkadienyl- oder Alkinylgruppe mit vorzugsweise 1 bis 24 Kohlenstoffatomen, wobei die Kohlenwasserstoffkette gegebenenfalls durch eine funktionelle Gruppe unterbrochen sein und/oder Träger von Substituenten sein kann;
- eine lineare oder verzweigte, gesättigte oder ungesättigte, acyclische aliphatische Gruppe, die Träger eines gegebenenfalls substituierten ringförmigen Substituenten sein kann, wobei die acyclische Gruppe über eine Valenzbindung, ein Heteroatom oder eine funktionelle Gruppe mit dem Ring verbunden sein kann;
- eine carbocyclische Gruppe, die gesättigt ist oder 1 oder 2 ungesättigte Bindungen im Ring enthält, mit im allgemeinen 3 bis 7 Kohlenstoffatomen, vorzugsweise 6 Kohlenstoffatomen im Ring, wobei der Ring substituiert sein kann.

**11.** Verfahren nach Anspruch 9 oder 10, **dadurch gekennzeichnet, daß** das Alkanol ein Terpenalkohol der Formel (IIa) ist:

$$T\text{-}OH \qquad (IIa)$$

wobei in der Formel (IIa):

- T den Rest eines Terpenalkohols mit einer Kohlenstoffanzahl mit einem Vielfachen von 5 darstellt.

**12.** Verfahren nach Anspruch 11, **dadurch gekennzeichnet, daß** der eingesetzte Terpenalkohol der allgemeinen Formel (IIa) entspricht, wobei der Rest T eine Kohlenwasserstoffgruppe mit 5 bis 40 Kohlenstoffatomen darstellt, insbesondere eine lineare oder verzweigte, gesättigte oder ungesättigte, aliphatische Gruppe oder eine monocyclische oder polycyclische, gesättigte, ungesättigte oder aromatische cycloaliphatische Gruppe, die Ringe mit 3 bis 8 Kohlenstoffatomen umfaßt.

**13.** Verfahren nach Anspruch 11 oder 12, **dadurch gekennzeichnet, daß** der Rest T den Rest:

- eines linearen oder verzweigten, gesättigten oder ungesättigten, aliphatischen Terpenalkohols,
- eines gesättigten, ungesättigten oder aromatischen, monocyclischen cycloaliphatischen Terpenalkohols,
- eines polycyclischen cycloaliphatischen Terpenalkohols, der mindestens zwei gesättigte und/oder ungesättigte Carbozyklen umfaßt,

darstellt.

**14.** Verfahren nach Anspruch 13, **dadurch gekennzeichnet, daß** der Alkohol der Formel (II) ausgewählt ist aus:

- Methanol,
- Ethanol,
- Trifluorethanol,
- Propanol oder Isopropylalkohol,
- Butanol, Isobutylalkohol, sek.-Butylalkohol oder tert.-Butylalkohol,
- Pentanol, Isopentylalkohol, sek.-Pentylalkohol und tert.-Pentylalkohol,
- 3-Chlorbut-2-en-1-ol,
- 2-Butin-1-ol,
- 3,7-Dimethyloct-6-en-1-ol,
- Chrysanthemumalkohol,
- 3,7-Dimethyloctanol,
- Geraniol,
- Linalool,
- Citronellol,
- Hydroxycitronellol,

- Nerol,
- Thymol,
- Menthol,
- Isoborneol,
- Verbenol.

**15.** Verfahren nach einem der Ansprüche 1 bis 14, **dadurch gekennzeichnet, daß** der Katalysator ein natürlicher oder synthetischer Zeolith ist.

**16.** Verfahren nach Anspruch 15, **dadurch gekennzeichnet, daß** der Zeolith ausgewählt ist aus:

- natürlichen Zeolithen, wie Chabazit, Clinoptilolit, Erionit, Mordenit, Phillipsit und Offretit,
- synthetischen Zeolithen mit eindimensionalem Netzwerk, wie Zeolith ZSM-4, Zeolith L, Zeolith ZSM-12, Zeolith ZSM-22, Zeolith ZSM-23 und Zeolith ZSM-48,
- Zeolithen mit zweidimensionalem Netzwerk, wie Mordenit und Ferrierit,
- Zeolithen mit dreidimensionalem Netzwerk, wie β-Zeolith, Zeolith Y, Zeolith X, Zeolith ZSM-5, Zeolith ZSM-11 und Offretit,
- mesoporösem Zeolith vom Typ MCM.

**17.** Verfahren nach Anspruch 16, **dadurch gekennzeichnet, daß** der Zeolith ein β-Zeolith ist.

**18.** Verfahren nach Anspruch 17, **dadurch gekennzeichnet, daß** der β-Zeolith ein Si/Al-Molverhältnis von mehr als 8, vorzugsweise zwischen 10 und 100, besonders bevorzugt von 12 bis 50, aufweist.

**19.** Verfahren nach einem der Ansprüche 15 bis 18, **dadurch gekennzeichnet, daß** der Zeolith allein oder in Mischung mit einer mineralischen Matrix eingesetzt ist, die vorzugsweise ausgewählt ist aus Metalloxiden, wie Aluminium-, Silicium- und/oder Zirkoniumoxiden, oder aber aus Tonen, insbesondere Kaolin, Talk oder Montmorillonit.

**20.** Verfahren nach einem der Ansprüche 1 bis 19, **dadurch gekennzeichnet, daß** das Verhältnis zwischen der Molzahl an Alkanol und der Molzahl an Benzylalkohol zwischen 1 und 30 variiert.

**21.** Verfahren nach einem der Ansprüche 1 bis 20, **dadurch gekennzeichnet, daß** die Menge an Katalysator, gewichtsbezogen auf das Reagenz im Unterschuß, 2 bis 50 %, vorzugsweise 5 bis 20 % beträgt.

**22.** Verfahren nach einem der Ansprüche 1 bis 21, **dadurch gekennzeichnet, daß** die Temperatur, bei der die Veretherungsreaktion durchgerührt wird, zwischen 50 °C und 200 °C, vorzugsweise zwischen 50 °C und 100 °C, liegt.

**23.** Verfahren nach einem der Ansprüche 1 bis 22, **dadurch gekennzeichnet, daß** die Reaktion unter Atmosphärendruck durchgeführt wird.

**24.** Verfahren nach einem der Ansprüche 1 bis 23, **dadurch gekennzeichnet, daß** die Verweildauer des Materialflusses im Katalysatorbett zwischen 15 Minuten und 10 Stunden, vorzugsweise zwischen 30 Minuten und 5 Stunden, variiert.

**25.** Verfahren nach einem der Ansprüche 1 bis 24, **dadurch gekennzeichnet, daß** man am Ende der Reaktion eine flüssige Phase erhält, die den veretherten Benzylalkohol umfaßt, der auf herkömmliche Weise erhalten werden kann.

**26.** Cyclohexyl-4-hydroxy-3-methoxybenzylether.

**27.** Verwendung von Cyclohexyl-4-hydroxy-3-methoxybenzylether als Duftstoffbestandteil (Parfümierbestandteil) auf dem Gebiet der Kosmetikindustrie/Parfümeriewaren.

**28.** Verfahren zur Herstellung von Duftstoffzusammensetzungen, Duftstoffsubstanzen und Duftstoffprodukten für die Kosmetikindustrie/Parfümeriewaren, **dadurch gekennzeichnet, daß** man zu den üblichen Bestandteilen dieser Zusammensetzungen, Substanzen und Endprodukte eine wirksame Menge an Cyclohexyl-4-hydroxy-3-methoxybenzylether hinzugibt.

**29.** Duftstoffzusammensetzungen, Duftstoffsubstanzen und Duftstoffprodukte, **dadurch gekennzeichnet, daß** sie als Aktivstoff mit Einfluß auf den Duft eine wirksame Menge an Cyclohexyl-4-hydroxy-3-methoxybenzylether enthalten.

**30.** Durftstoffartikel nach Anspruch 29 in Form eines Duftstoffs, von Eau-de-Toilette, After-Shave-Lotionen, Parfümen, Seifen, Bade- oder Duschgelen, desodorierenden Produkten oder Antitranspirantien, Shampoos oder Haarpflegeprodukten, Talkum oder Pudern aller Art, Desodorantien für die Raumluft, Pflegeprodukten aller Art und Waschmittel- und Weichspülzusammensetzungen fiir Textilien.